# EUROPEAN PATENT APPLICATION

(11) **EP 4 786 954 A1**
(43) Date of publication of application: **05.08.2026**
(21) Application number: 25155113.1
(22) Date of filing: 30.01.2025
(51) Int. Cl.: G01N 15/14, B01L 3/00

(54) **GAMMA-STERILIZABLE CHIP, PARTICULARLY FOR IMPEDANCE FLOW CYTOMETRY**

(71) Applicant: Mettler-Toledo GmbH, 8606 Greifensee (CH)
(72) Inventor: Bergström, Pär, Meilen (CH); Hertel, Martin, Steinen (DE); Tarancon, Antonio, Brugg (CH)
(74) Representative: Mettler-Toledo

(57) **Abstract**

The present invention relates to a sensor cartridge (1) for inline measurement of a fluid (2), comprising: a body (10) having an interface section (11) configured to be connected to a sensor head, the interface section (11) comprising a measuring channel (12) for accommodating the fluid (2) to be measured, a pump (13) accommodated in the body (10), the pump (13) comprising an actuating member (130) being configured to be actuated by the sensor head when the interface section (11) is connected to the sensor head to pump the fluid (2) to be analyzed through the measuring channel (12), wherein the body (10) further comprises an in- and outflow section (14) connected to the interface section (11), wherein the in- and outflow section (14) comprises an inflow channel (15) and an outflow channel (16), the inflow channel (15) being in flow connection with the outflow channel (16) via the measuring channel (12) and the pump (13) so that the fluid (2) to be measured can be pumped from the inflow channel (15) via the measuring channel (12) to the outflow channel (16) to enable an inline measurement of the fluid (2).

## Description

The present invention relates to a sensor cartridge for inline measurement of a fluid, a system comprising a sensor cartridge according to the present invention and a sensor head, and to a method for inline measuring of a fluid.

Particularly, such sensor cartridges can be used to perform flow cytometry, particularly impedance flow cytometry.

Flow cytometry allows to analyze and quantify the characteristics of cells or particles in a fluid. It is widely employed in various fields, including immunology, hematology, microbiology, etc. The basic principle of flow cytometry can involve the passage of individual particles/cells through a laser beam, and the measurement of light scatter and fluorescence emitted by the cells.

Impedance flow cytometry is a specific type of flow cytometry that measures changes in electrical impedance as said particles or cells pass through a measuring channel, e.g. a small aperture. Here, electrodes can be arranged on either side of the channel/aperture, and a detector can be used to measure the changes in electrical impedance as the individual particle or cell passes the electrodes. Particularly, the channel is configured in a manner that the particles or cells flow through the channel in a single file so that ideally only one particle or cell passes through at a time. As each particle or cell passes through the channel, it changes the electric field between the electrodes. This change in the electrical impedance is directly proportional to the particle or cell volume. Larger particles or cells cause a greater change in impedance compared to smaller cells. The changes in electrical impedance can be recorded and analyzed to determine various characteristics of the particles or cells, such as cell size, granularity, and count.

EP3914393A1 discloses a microfluidic chip system (e.g. a cytometry system) with on-chip impedance-based measurement and quantification. An electronic circuit portion comprises a printed circuit board configured with an insert for the placement of a microfluidic chip portion.

The electronic circuit portion may include electronics for a signal generator, controller, and impedance detector that can interface to electrodes on the microfluidic chip.

Furthermore, also US2003178310A1 discloses an "on-chip" detection with integrated electrodes.

Further, US2015024373A1 relates to a microfluidic chip that orients and isolates components in a sample fluid mixture by two-step focusing.

Furthermore, US2003152487A1 discloses a sample analysis instrument for use with a fluidic analysis cartridge. The instrument includes a cartridge holder, a flow cytometric measuring apparatus positioned for optical coupling with a flow cytometric measuring region on the cartridge. The cartridge holder can include pump mechanisms to couple with pump interfaces on the cartridge and valve mechanisms to couple with valve interfaces on the cartridge.

Based on the above, the problem to be solved by the present invention is to provide an improved sensor cartridge that allows for inline measuring of a fluid comprising particles or cells in a user-friendly manner and particularly facilitates sterilization of the sensor cartridge for waste reduction.

This problem is solved by a sensor cartridge for inline measurement of a fluid, a system comprising such a sensor cartridge and a sensor head, as well as by a method for inline measuring of a fluid.

In the context of this invention, "inline" measurement refers to a measurement directly in the processes respectively in its immediate vicinity: There is no need for the user to provide additional hardware to extract a sample or to create a bypass.

Preferred embodiments of theses aspects of the present invention are stated in the corresponding dependent claims and are described below.

According to claim 1, a sensor cartridge for inline measurement of a fluid containing particles or cells is disclosed, comprising:
- a body having an interface section configured to be connected to a sensor head, the interface section comprising a measuring channel for accommodating a volume of the fluid to be measured,
- a pump accommodated in the body, the pump comprising an actuating member being configured to be actuated by the sensor head when the interface section is connected to the sensor head to pump the fluid to be analyzed through the measuring channel, wherein
- the body further comprises an in- and outflow section connected to the interface section, wherein the in- and outflow section comprises an inflow channel and an outflow channel, the inflow channel being in flow connection with the outflow channel via the measuring channel and the pump so that the fluid to be measured can be pumped from the inflow channel via the measuring channel to the outflow channel, particularly to enable an inline measurement of the fluid.

Generally, in the framework of the present invention, the fluid carries the particles / cells to be measured, wherein the fluid is preferably a liquid.

Furthermore, in a particular embodiment of the sensor cartridge, the interface section is configured to be inserted into a slot of the sensor head to connect the sensor cartridge to the sensor head. Particularly, in one possible configuration, instead of inserting the sensor cartridge, the latter may also be pressed onto a receiving section of the sensor head by means of a clip for example.

Further, as exemplified in one embodiment of the sensor cartridge, the inline measurement is a cytometric measurement, and the measuring channel is configured to accommodate a fluid comprising particles or cells and the pump is particularly configured to pump said fluid comprising said particles or cells.

According to an embodiment of the sensor cartridge, the latter is a disposable unit. In the context of the present invention, the notion of a disposable unit refers to a unit designed for a single use and intended to be discarded after this single use. Thus, such a unit is not meant for reuse or extended lifespan. Advantageously, the fact that the sensor cartridge can be a disposable unit enhances convenience, reduces the risk of contamination, and can simplify the measurement process that utilizes the sensor cartridge.

According to yet another embodiment of the sensor cartridge, the body of the sensor cartridge is gamma-sterilizable, i.e. sterilizable by means of gamma radiation. Such a process is commonly used to sterilize items such as laboratory equipment, medical devices, pharmaceuticals, and other products. Gamma sterilization can typically involve exposing the respective item to ionizing gamma radiation, e.g. from a radioactive isotope such as cobalt-60 or cesium-137. The high-energy gamma rays disrupt the DNA and other cellular structures of microorganisms, rendering them unable to reproduce and thereby sterilizing the respective item. Preferably, the sensor cartridge is gamma-sterilizable, i.e. sterilizable by means of gamma radiation.

According to an alternative embodiment, the sensor cartridge is sterile (e.g. by sterilizing an intermediate product of the sensor cartridge during a manufacturing process of the sensor cartridge).

Further, in an embodiment of the sensor cartridge, the interface section is formed as a flat plate. Particularly, in the context of the present invention, the notion "flat" particularly means that the interface section comprises a thickness in a thickness direction, which thickness is smaller than the dimensions of the interface section in two perpendicular directions that are orthogonal to the thickness direction, respectively. In a particular embodiment, the interface section comprises a top side and a bottom side which are spaced apart from one another by said thickness in said thickness direction, and each extend orthogonal to the thickness direction. Particularly, the top side and the bottom side extend parallel to one another.

In accordance with a further embodiment of the sensor cartridge, the in- and outflow section comprises an elongated and/or cylindrical shape.

Particularly, in an embodiment, the in- and outflow section comprises a proximal end connected to the interface section and an opposing distal end forming a face side of the in- and outflow section, wherein an inlet to the inflow channel is arranged on the face side, and wherein an outlet of the outflow channel is arranged on the face side besides the inlet. Particularly, inlet and outlet can be arranged on opposite regions of the face side of the in- and outflow section.

According to a further embodiment of the sensor cartridge, the proximal end is integrally connected to the interface section of the body of the sensor cartridge.

Furthermore, in an embodiment of the sensor cartridge, the inflow channel extends parallel to the outflow channel.

According to yet another embodiment of the sensor cartridge, the pump is a membrane pump, wherein said actuating member is an elastically deformable membrane. Alternatively, the pump can be a gear pump, or a peristaltic pump. In the case of a gear pump, said actuating member comprises a gear. In the case of a peristaltic pump, said actuating member comprises an elastically deformable membrane.

Furthermore, in a particular embodiment of the sensor cartridge, the in- and outflow section and/or the interface section is/are formed out of or comprise(s) one of the following materials: a glass, acrylic glass, polyether ether ketone (PEEK), high-density polyethylene (HDPE). Particularly, the density of HDPE ranges from 930 kg/m³ to 970 kg/m³. The standard method to test plastic density and which may also be employed in the context of the present invention is ISO 1183 part 2 (gradient columns), alternatively ISO 1183 part 1; both in the version of 2019-03. Particularly, suitable materials for the in- and outflow section and/or the interface section of the sensor cartridge comprise chemical compatibility with typical bio-process media used with the sensor cartridge. A typical bio-process media is for example a culture media comprising water, salts, and a carbon source such as glucose.

Furthermore, in an embodiment, the interface section of the sensor cartridge comprises a transparent window, particularly in form of a transparent portion of the interface section, in the vicinity of the measuring channel for optically observing the fluid to be measured (e.g. for conducting cytometry).

In a particular embodiment of the invention, the sensor cartridge comprises at least a pair of electrodes arranged on the interface section on opposing sides of the measuring channel to generate an electric field in the vicinity of the fluid to be measured.

Particularly, this facilitates the sensor cartridge to perform impedance flow cytometry. Preferably, the sensor cartridge comprises two of such pairs of electrodes arranged along the measuring channel behind each other suitable to perform impedance flow cytometry using the differential measurement of the pair of electrodes.

Particularly, the at least one pair of electrodes facilitates the sensor cartridge to perform electrophoresis.

Particularly, further pairs of electrodes facilitate the sensor cartridge to shield the different electrode pairs from each other.

Particularly, in an embodiment, the sensor cartridge comprises at least a pair of focusing electrodes arranged on the interface section on opposing sides of the measuring channel to align the cells or particles to be measured in the measurement channel.

According to yet another embodiment of the sensor cartridge, the latter comprises electrical contacts for electrically connecting electrodes arranged on the interface section to an electronic unit of the sensor head, when the interface section is connected to the sensor head (particularly inserted into the sensor head).

Furthermore, in an embodiment of the sensor cartridge, the measuring channel of the sensor cartridge comprises a first measuring section and a second measuring section.

Particularly, in an embodiment, the first measuring section is arranged upstream of the pump between the inflow channel and the pump, and wherein the second measuring section is arranged downstream of the first measuring section and upstream of the pump, or wherein the second measuring section is arranged downstream the pump between the pump and the outflow channel. Particularly, having both measuring sections upstream of the pump allows to measure cells in the fluid before passing the pump thus reducing the risk of measuring cells that have been damaged by the pump upon passing the latter.

In an embodiment, the sensor cartridge is configured for measuring the fluid and/or the particles and/or cells in the fluid using a first measurement method and the first measuring section, adapted for this first measurement method and the sensor cartridge is configured for conducting an alternative measurement of the fluid and/or the particles and/or cells in the fluid using the second measuring section, adapted for this second measurement method. The adaption of the measuring section to a particular measurement method comprises in particular one or more of the following features or a combination of them: one or more windows which are shaped to act as lenses and/or made of a specific material or coated to be transparent, reflecting or absorbing to specific wavelengths; electrodes of suitable shape, sizes and/or arrangements to create, change and/or measure electric fields; spots comprising coatings and/or dyes, which react to a property to be measured.

In a further embodiment, the sensor cartridge is configured for impedance flow cytometry using the first measuring section, and wherein particularly the sensor cartridge is configured for conducting an alternative measurement using the second measuring section, wherein particularly said alternative measurement is an optical measurement, particularly optical microscopy.

According to a further aspect of the present invention, a system comprising a sensor cartridge according to the present invention and a sensor head is disclosed, the sensor head comprising a receiving section, preferably in form of a slot, wherein the interface section of the sensor cartridge is configured to be connected to the receiving section of the sensor head so that the inflow section protrudes from the sensor head. Particularly the interface section of the sensor cartridge is configured to be inserted into the slot to connect the sensor cartridge to the receiving section. A corresponding insertion direction can coincide with a longitudinal axis along which the in- and outflow section and particularly the in- and outflow channels extend. Particularly, in an embodiment of the system, the sensor head comprises at least one measurement instrument, arranged in the sensor head and intended to observe a property of the fluid in the measuring channel.

Preferably one of the at least one measurement instruments is at least a part of a microscope for optically observing cells and particles in the fluid and/or the fluid in the measuring channel, most preferable through a window in the body of the sensor cartridge. In such an embodiment, the window may be formed by a lens and can thereby be part of the measurement instrument, in particular part of the microscope. Preferably, a part of a microscope comprised in the sensor head and a part of a microscope formed by a lens acting as a window in the body of a sensor cartridge can be combined by connecting the sensor cartridge to the receiving section to form together a microscope.

In a preferred embodiment, a second window may be formed, preferably on the opposite side of the measuring channel or adjacent to the first window, and this second window is preferably used to supply illumination to part of measuring channel observed by the microscope. Preferably, the at least one measurement instrument or the parts of it which are included in the sensor head is mounted and configured such as to be aligned relative to the sensor cartridge when the sensor cartridge is connected the receiving section, to allow the desired measurements. If needed, this can be archived for example by spring loading the at least one measurement instruments or the parts of it included in the sensor head and by proving both, the sensor cartridge and the measurement instruments or parts of it with complementary alignment structures such as for example, a ball pin and a groove.

Particularly, in an embodiment of the system, the sensor head comprises an electronic unit configured to be electrically connected to either the sensor cartridge and/or to additional measurement instruments, arranged in the sensor head and intended to observe a property of the fluid in the measuring channel, when the sensor cartridge is connected the receiving section, particularly inserted in said slot, wherein particularly in an embodiment of the system, the electronic unit is configured to process signals provided by the sensor cartridge to evaluate an impedance or another property of particles or cells (e.g. for conducting impedance flow cytometry or electrophoresis).

Preferably, the electronic unit is configured to be electrically connected to the electrical contacts of the sensor cartridge. Alternatively, according to an embodiment of the system, the electronic unit can be configured to pre-process signals provided by the sensor cartridge and to provide the pre-processed signals to a further device for analyzing the pre-processed signals, particularly for evaluating an impedance (e.g. for conducting impedance flow cytometry or electrophoresis).

Particularly, in an embodiment of the system, the electronic unit is configured to provide signals to the electrical contacts of the sensor cartridge to create electric fields needed for the intended measurements, in particular to deliver a desired voltage or current signal to the electrodes to conduct the measurement and/or to connect electrodes to a common ground:
For example, for conducting an impedance measurement for an impedance flow cytometry, a AC voltage signal of a desired frequency or of a set of frequencies ("driving frequency") can be applied to one, two or more pairs of electrodes and the phase shift between this driving frequency and the observed frequency and/or between the observed frequencies at the respective pairs can be evaluated to obtain information about the presence and/or the properties of the particle or cell passing the electric field created by the respective pair of electrodes. For this example, the sensor cartridge comprises the one, two or more pairs of electrodes and the sensor head comprises an electronic unit comprising contacts to the different electrodes provided on the sensor cartridge.

For example, for conducting electrophoresis, a DC voltage or an AC voltage of a low frequency can be applied to a pair of electrodes and the motion of the cell or a particle in response to this electric field is observed by optical means through the transparent window to be evaluated to obtain information about the presence and/or the properties of the particle or cell passing the field created by the pair of electrodes. For this example, the sensor cartridge comprises the pair of electrodes and an optical window to observe the measuring channel in the region where it passes through the electric field created by the pair of electrodes and the sensor head comprises an electronic unit comprising contacts to the electrodes provided on the sensor cartridge and the sensor head comprises further an optical means to observe the measuring channel in the region where it passes through the electric field created by the pair of electrodes through the optical window.

Particularly, in an embodiment of the system, the sensor head comprises a first measurement instrument and either a second measurement instrument or an electronic unit suitable to provide signals to the electrical contacts on the sensor cartridge. In this system, the sensor cartridge comprises a first measuring section adapted to the first measurement instrument and a second measuring section adapted to the second measurement instruments and/or comprising electrodes and electrical contacts to these electrodes. This embodiment allows to observe the same flow and/or particles and/or cells with at least two different measurement methods: One using a first measurement instrument and one using a second measurement instrument and/or electrodes.

The resulting dataset of the same flow and/or particles and/or cells being observed with the different measurement methods is preferably used to create a training set for a machine learning system. A first set of these different measurement methods, -the first set can comprise one or more measurement methods-, creates measurement results which are evaluated to become labels. These labels are assigned to measurement results of a second set of these different measurement methods, -the second set can comprise one or more measurement methods. The assignment of the labels to the measurement results of the second set is done automatically and preferably based on a time stamp: Due to the integration of pump driving the flow and the measurement instruments and/or electrodes in the same system, the time differences between the observations of the same flow and/or particles and/or cells by the different measurement methods are precisely know.

This training set is preferably subsequently used by a machine learning system or an artificial intelligence (Al) to derive a trained Al model which automatically assigns the property described by the values of the labels to measurement results obtained by at least one of measurement methods of the second set.

In another embodiment, the training set is evaluated to derive or test an algorithm, which when executed on a computing device automatically assigns the property described by the values of the labels to measurement results obtained by at least one of measurement methods of the second set.

In a preferred embodiment, the measurement method of the second set is an optical method, creating images, most preferably microscopic images. The trained Al model or the algorithm allows in this embodiment preferably an object recognition and/or object segmentation on said images and preferably categorizes the images.

In particular, the measurement method of the first set is impedance spectroscopy, and the measurement method of the second set is microscopy. In this example, the impedance spectroscopy is evaluated to evaluate the presence of a cell and its viability status. The presence of a cell and/or its viability is used a label for the corresponding microscopic image.

If, for example, the impedance spectroscopy is conducted, symmetrically arranged along the measuring channel, before and behind a window through which the microscopic image is taken, the impedance spectroscopy measurements of the first set of electrodes at time t - Δ t and the impedance spectroscopy measurements of the second set of electrodes at time t + Δ t can be evaluate to create the label for the microscopic image taken at time t. The resulting training set comprises thereby a set of microscopic images labeled with the presence or absence of cells and their respective viability status. This training set is entered in a machine learning system or an artificial intelligence (Al) to create a trained model to identify cells and their viability on microscopic images. According to yet another embodiment of the system, the sensor head comprises an actuator configured to actuate the pump of the sensor cartridge, particularly by deforming said membrane forming thereby a membrane or a peristatic pump or by driving at least one gear of a gear pump.

Furthermore, according to an embodiment of the system, the latter comprises an assembly configured to position and to releasably lock the sensor cartridge and the sensor head with respect to each other, wherein particularly the assembly is configured to provide a form-fitting seat (e.g. for the sensor cartridge) to position the sensor cartridge and the sensor head with respect to each other, and/or wherein particularly the assembly comprises a releasable lock configured to releasably lock the sensor cartridge relative to the sensor head in said desired position (as e.g. provided by said seat). Particularly, said releasable lock can comprises a latch, particularly a movable latch. Said assembly is a positioning and/or locking assembly. According to a further embodiment of the system, the sensor head and/or the sensor cartridge comprise a coupling device configured to establish a particularly fluid-tight connection with a measurement port, particularly a measurement port in a fluid line, or of fluid reservoir, or of a reactor.

In an embodiment, the coupling device comprises a circumferential seal, wherein particularly the seal is an O-ring, and wherein particularly the seal is configured to form a seal with an inner surface of the measurement port when the in- and outflow section is arranged in said measurement port. Particularly, in an embodiment, the in- and outflow section comprises the coupling device or the latter is arranged thereon.

Furthermore, according to yet another embodiment of the system, the coupling device comprises one of: a male thread suitable to connect with a corresponding female thread on an inner side of the measurement port, a union nut, a female part of a bayonet mount, a retainer, the retainer being particularly configured to engage with a measurement port in form of an Eldon-James port.

According to yet another embodiment of the system, the system comprises a container for accommodating a fluid to be measured, wherein - as described above - said container can be a fluid line, a fluid reservoir or a reactor, wherein the sensor cartridge is part of a closed fluid system comprising the container and the sensor cartridge, and wherein the sensor head is separated from said fluid system in a liquid tight fashion (e.g. by the sensor cartridge being coupled in a liquid tight fashion to the container by means of said coupling device such that inflow, measuring and outflow channel form a conduit which only opens to the container, while the sensor head being coupled to the sensor cartridge). Particularly, the sensor cartridge is part of the fluid system, while the sensor head does not touch the inside of the container. Thus, particularly, the sensor head does not have to be sterile, only the sensor cartridge.

According to a further aspect of the present invention, a method for inline measuring of a fluid using a system according to the present invention is disclosed, wherein the in- and outflow section of the sensor cartridge being connected to the sensor head is arranged in flow connection with a flow of a fluid to be measured and a partial flow of the fluid is pumped by the pump into the sensor cartridge via the inflow channel, wherein the partial flow is measured in the measuring channel of the sensor cartridge with help of the sensor head, and wherein the partial flow is discharged through the outflow channel and united with the flow of the fluid. Further features and advantages of the present inventions as well as embodiments of the present invention shall be described in the following with reference to the Figures, wherein
- Fig. 1A: shows a perspective view onto an embodiment of a sensor cartridge according to the present invention,
- Fig. 1B: shows a partial cross-section of an embodiment of a sensor cartridge according to the present invention.
- Fig. 2: shows a perspective view of an embodiment of a system according to the present invention, wherein the system comprises a sensor cartridge according to the present invention (e.g. as shown in Fig. 1) and a sensor head, wherein Fig. 2A shows the sensor cartridge being released from the sensor head,
- Fig. 3A: shows the system with the sensor cartridge being inserted into the sensor head,
- Fig. 3B: shows a schematic cross-section of an embodiment of an assembly of the system shown in Figs. 2 and 3A allowing to position and particularly to releasably lock the sensor cartridge with respect to the sensor head when being inserted in the sensor head,
- Fig. 4: shows an embodiment of the system according to the invention as e.g. shown in Figs. 2 to 3B, wherein the sensor cartridge is positioned in a measurement port of a container of the system, wherein - as an example - the container is a fluid line guiding the fluid to be measured,
- Fig. 5: shows a detail of an embodiment of a coupling device of the system that allows to couple the sensor cartridge to a measurement port of a container (e.g. the fluid line of Fig. 4) in a fluid-tight fashion, and
- Fig. 6: shows a detail of a further embodiment of a coupling device of the system allowing to couple the sensor cartridge to a measurement port of a container (e.g. the fluid line of Fig. 4) in a fluid-tight fashion.

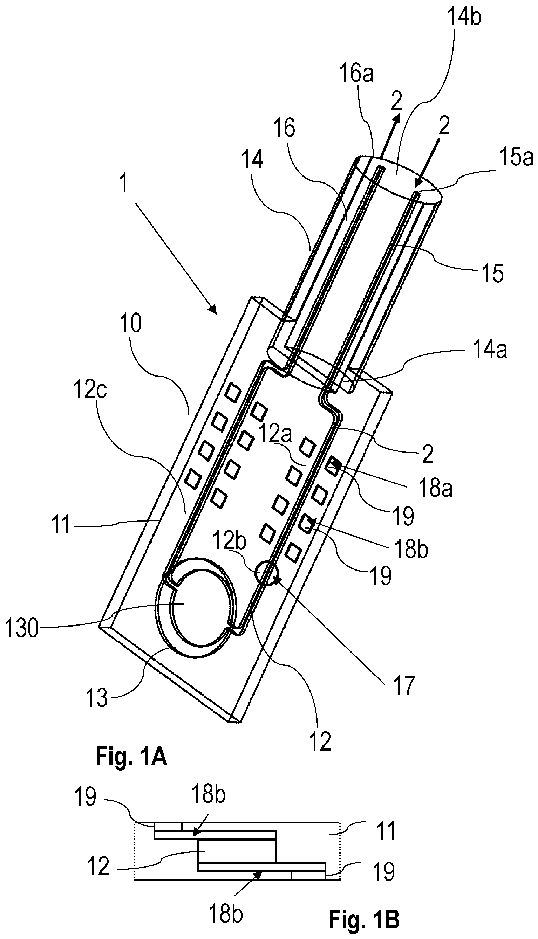

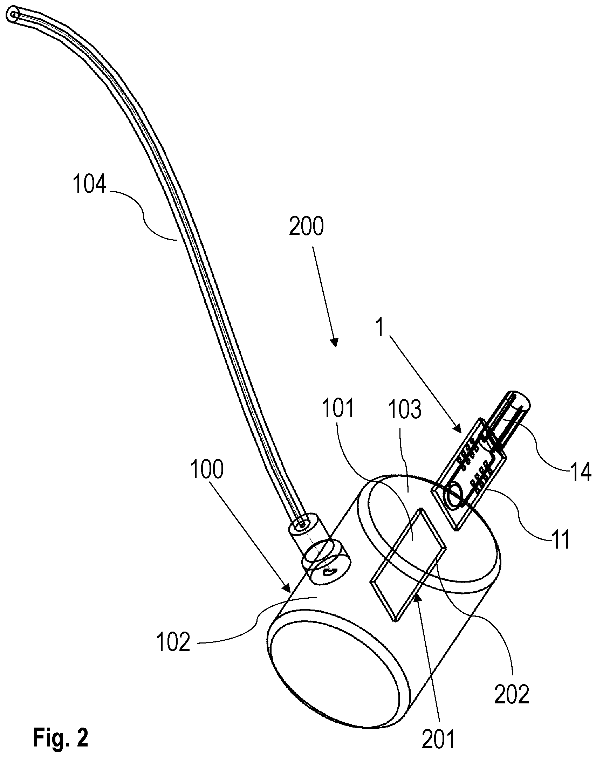

Fig. 1A shows an embodiment of a sensor cartridge 1 for inline measurement of a fluid 2. As indicated in Fig.1A, the sensor cartridge comprises 1 a body 10 having an interface section 11 that can be shaped like a flat, e.g. rectangular, plate and is configured to be connected to a sensor head 100 (not shown in Fig. 1A, cf. Figs. 2 to 4). The interface section 11 encloses a measuring channel 12 for guiding a flow of the fluid 2 to be measured. To this end, the sensor cartridge 1 further comprises a pump 13 arranged on the body 10, wherein the pump 13 comprises an actuating member 130, e.g., in form of a membrane 130, that is configured to be actuated by the sensor head 100 when the interface section 11 is connected to the sensor head 100, to pump the fluid 2 to be analyzed through the measuring channel 12. Particularly, the sensor head 100 can comprise an actuator that is operatively coupled to the membrane 130 to move the latter for pumping the fluid 2 through the measuring channel 12. Furthermore, the body 10 comprises an in- and outflow section 14 connected to the interface section 11, wherein the in- and outflow section 14 comprises an inflow channel 15 and an outflow channel 16, the inflow channel 15 being in flow connection with the outflow channel 16 via the measuring channel 12 and the pump 13 so that the fluid 2 to be measured can be pumped from the inflow channel 15 via the measuring channel 12 to the outflow channel 16. Particularly, the in- and outflow section 14 can comprise a proximal end 14a connected to the interface section 11 and an opposing distal end 14b forming a face side of the in- and outflow section 14, wherein an inlet 15a to the inflow channel 15 is arranged on the face side 14b, and wherein an outlet 16a of the outflow channel 16 is arranged on the face side 14b besides the inlet 15a. Furthermore, the inflow channel 15 can extend parallel to the outflow channel 16. Particularly, the above-described configuration of the in- and outflow section 14 supports the possibility to perform inline measurements in an advantageous fashion as the fluid 2 to be analyzed can be easily drawn off a container (such as a fluid line or a reactor) through the in- and outflow section 11 and can be discharged back into the container via the same in- and outflow section 11. Said in- and outflow section 14 can comprise an elongated and particularly cylindrical shape and protrudes from the interface section 11 so that it can be easily brought into contact with the fluid 2 via a relatively small measurement port of the container and effectively sealed with respect to the measurement port of said container.

The interface section 11 of the sensor cartridge 1 houses the measuring channel 12, the pump 13 and, according to an embodiment, electrodes 18a, 18b and corresponding electrical contacts 19 that allow the sensor cartridge 1 to be electrically contacted by the sensor head 100. The electrodes 18a, 18b arranged in pairs on either side of the measuring channel 12 allow the sensor cartridge 1 to perform impedance flow cytometry. Particularly, the sensor cartridge 1 can comprises at least a pair of focusing electrodes 18a arranged on the interface section 11 on opposing sides of the measuring channel 12 to generate an electric field in the vicinity of the fluid 2 for interaction with the particles/cells to be measured and to align them in the measuring channel 12. Furthermore, the sensor cartridge 1 can comprise at least a pair of electrodes 18b arranged on the interface section 11 on opposing sides of the measuring channel 12 for acquiring a signal from which an impedance of the particles or cells can be derived.

In order to facilitate different measuring techniques, the measuring channel 12 of the sensor cartridge 1 can comprise multiple measuring sections such as a first measuring section 12a and a succeeding second measuring section 12b wherein both measuring sections are arranged upstream the pump 13 which has the benefit that cells contained in the liquid are measured in said sections 12a, 12b before passing the pump 13. Particularly, impedance flow cytometry may be conducted in the first measuring section 12a, while a different measurement, e.g., an optical measurement, can be performed in the second measuring section 12b of the measuring channel 12. However, the second or a further measuring section 12c may also be arranged downstream the pump 13 instead (see also above). For conducting optical measurements, the interface section 11 of the sensor cartridge 1 can comprises a transparent window 17 in the vicinity of the measuring channel 12. Particularly, the interface section 11 may or may be at least partially formed out of a transparent material.

Furthermore, the afore-described partition of the sensor cartridge 1, preferably along its longitudinal axis, into the in- and outflow section 14 on one end and the interface section 11 on the other end, allows to use the interface section 11 for contacting the sensor head 100 in a safe and reliable manner, e.g. by connection of the interface section 11 with a receiving section 101 of the sensor head 100. Particularly, the receiving section 101 can be formed by a slot 101 of a housing 102 of the sensor head 100 allowing insertion of the interface section 14 into the slot 101 to establish all necessary electrical connections between the sensor head 100 and the sensor cartridge 1. Particularly, connecting the sensor cartridge 1 to the receiving section 101 as intended (e.g. by way of insertion of the sensor cartridge 1 into the slot 101) can also align the actuating member 130 of the pump 13 with an actuator comprised by the sensor head 100 to allow actuation of the pump 13.

Fig. 1B shows a cross-section of the sensor cartridge 1 perpendicular to the extent of the measuring channel 12 through a pair of electrodes 18b: The electrodes 18b are arranged on opposite sides of the measuring channel 12 and their voltage is during a measurement either controlled or measured by the sensor head contacting them via the corresponding electrical contacts 19. The electrical contacts 19 are in electrical contact with the respective electrode 18b.

Embodiments of such a system 200, comprising the sensor cartridge 1 and the sensor head 100 are shown in Figs. 2 to 6.

Fig. 2 shows the sensor head 100 and the sensor cartridge 1 before insertion of the sensor cartridge 1 into the receiving section / slot 101. The sensor head 100 can comprise an e.g. cylindrical housing 102 having a face side 103 that provided access to the slot 101.

Furthermore, electrical energy as well as communication with further devices can be provided via a lead 104 that is connected to the sensor head 100. Thus, analysis of signals measured by the sensor cartridge 1 may also be performed by a remote device connected to the sensor head 100 via lead 104.

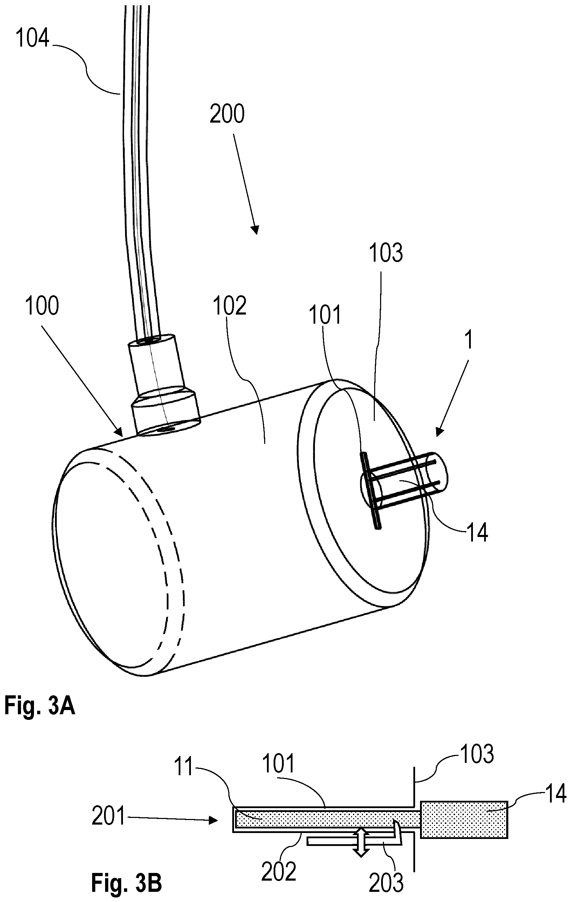

Fig. 3A shows the sensor cartridge 1 with the interface section 11 being inserted into the slot 101 of the sensor head. Particularly, as schematically indicated in Fig. 3B, the system 200, particularly the sensor head 100, can comprises an assembly 201 configured to position and/or to releasably lock the sensor cartridge 1 and the sensor head 100 with respect to each other.

To this end, the assembly 201 can provide a form-fitting seat 202 to position the sensor cartridge 1 and the sensor head 100 with respect to each other. Particularly, the seat 202 can be defined by the slot 101 and/or a guiding structure arranged in the slot 101. Further, the assembly 201 can comprise a releasable lock 203 that is configured to releasably lock the sensor cartridge 1 relative to the sensor head 100, particularly in said position provided by the seat 202. As indicated in Fig. 3B, the lock 203 can comprise a movable latch that may prevent the sensor cartridge from being pulled out of the slot 101 when the latch is engaged with a portion of the interface section 11.

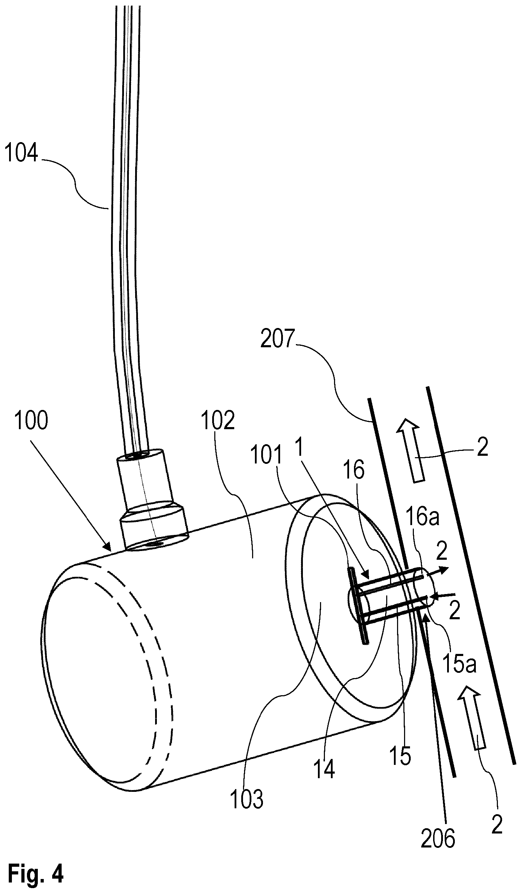

Furthermore, Fig. 4 shows an example of how the in- and outflow section 14 of the sensor cartridge 1 may be connected to a container 207, here a fluid line 207 guiding the fluid 2, via a measurement port 206 of the container 207. Particularly, the in- and outflow section 14 is inserted with its distal end 14b ahead into the measurement port 206 thus establishing a flow connection between the fluid line 207 and inlet 15a as well as outlet 16a of the in- and outflow section 14 of the sensor cartridge 1.

Particularly, in order to achieve a fluid-tight, i.e. sealed, connection between the in- and outflow section 14 and the measurement port 206, the sensor head 100 and/or the sensor cartridge 1 can comprise a coupling device 205 configured to establish a fluid-tight connection with the measurement port 206 of said container/fluid line 207.

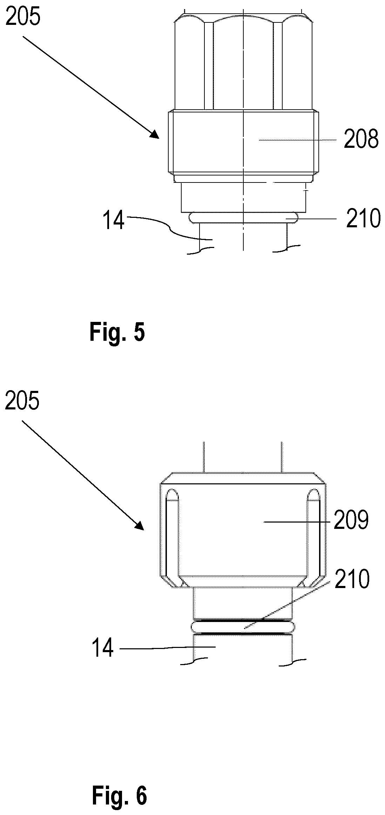

According to an embodiment shown in Fig. 5, the coupling device 205 can comprise a male (i.e. outer) thread 208 configured to engage with a corresponding female thread formed in the measurement port 206. The coupling device 205 can further comprise at least one seal 210, particularly an O-ring, arranged on the in- and outflow section 14 that may contact an inner side of the measurement port 206 to seal the connection between the in- and outflow section 14 of the sensor cartridge 1 and the measurement port 206.

Fig. 6 exemplifies an alternative embodiment of the coupling device 205, wherein here the coupling device 205 comprise a retainer 209 that can encompass a nozzle of the measurement port (e.g. Eldon-James port) 206. Also here, a seal 210 can be arranged on the in- and outflow section 14 of the sensor cartridge 1 that may contact an inner side of the measurement port 206 to seal the connection between the in- and outflow section 14 of the sensor cartridge 1 and the measurement port 206.

The present invention provides an advantageous sensor cartridge that enables inline measurements of fluids, particularly based on impedance flow cytometry. Particularly, the specific design of the sensor cartridge allows its in- and outflow section 14 to be brought in flow connection with a fluid to be measured in a convenient fashion (e.g. via a small measurement port) while guaranteeing efficient sealing of the connection between the in- and outflow section 14 of the sensor cartridge 1 and the container (e.g. fluid line, reactor and the like) containing / guiding the fluid to be measured. The interface section 11 from which the in- and outflow section 14 protrudes, allows easy and user-friendly operation of the sensor cartridge 1 with a corresponding sensor head 100, thereby ensuring proper electrical contact between the sensor cartridge 1 and the sensor head 100 as well as proper interaction with functions contained in the sensor head 100 such as the actuator of the pump 13. Particularly, as the sensor head 100 can be completely separated from the fluid, only the sensor cartridge 1 needs to be sterile.

## Claims

1. A sensor cartridge (1) for inline measurement of a fluid (2), comprising:
- a body (10) having an interface section (11) configured to be connected to a sensor head (100), the interface section (11) comprising a measuring channel (12) for accommodating a volume of the fluid (2) to be measured,
- a pump (13) accommodated in the body (10), the pump (13) comprising an actuating member (130) being configured to be actuated by the sensor head (100) when the interface section (11) is connected to the sensor head (100) to pump the fluid (2) to be analyzed through the measuring channel (12), wherein
- the body (10) further comprises an in- and outflow section (14) connected to the interface section (11), wherein the in- and outflow section (14) comprises an inflow channel (15) and an outflow channel (16), the inflow channel (15) being in flow connection with the outflow channel (16) via the measuring channel (12) and the pump (13) so that the fluid (2) to be measured can be pumped from the inflow channel (15) via the measuring channel (12) to the outflow channel (16) to enable an inline measurement of the fluid (2).

2. The sensor cartridge according to claim 1, wherein the inline measurement is a cytometric measurement, and the measuring channel (12) is configured to accommodate the fluid (2) comprising particles or cells and the pump (13) is configured to pump said fluid (2).

3. The sensor cartridge according to one of the preceding claims, wherein the body (10), preferably the sensor cartridge, is gamma-sterilizable.

4. The sensor cartridge according to one of the preceding claims, wherein the in- and outflow section (14) comprises a cylindrical shape.

5. The sensor cartridge according to one of the preceding claims, wherein the in- and outflow section (14) comprises a proximal end (14a) connected to the interface section (11) and an opposing distal end (14b) forming a face side of the in- and outflow section (14), wherein an inlet (15a) to the inflow channel (15) is arranged on the face side (14b) and wherein an outlet (16a) of the outflow channel (16) is arranged on the face side (14b) besides the inlet (15a).

6. The sensor cartridge according to one of the preceding claims, wherein the interface section (11) of the sensor cartridge (1) comprises a window (17) in the vicinity of the measuring channel (12) for optically observing the fluid (2) to be measured.

7. The sensor cartridge according to one of the preceding claims, wherein the sensor cartridge (1) comprises at least a pair of electrodes (18a, b) arranged on the interface section (11) on opposing sides of the measuring channel (12) to generate an electric field in the vicinity of the fluid (2) to be measured.

8. The sensor cartridge according to one of the preceding claims, wherein the sensor cartridge (1) comprises electrical contacts (19) for electrically connecting electrodes (18a, 18b) arranged on the interface section to an electronic unit of the sensor head, when the interface section (11) is connected to the sensor head (100).

9. The sensor cartridge according to one of the preceding claims, wherein measuring channel (12) of the sensor cartridge (1) comprises a first measuring section (12a) and a second measuring section (12b, 12c).

10. The sensor cartridge according to claim 9, wherein the sensor cartridge (1) is configured for impedance flow cytometry using the first measuring section (12a), and wherein particularly the sensor cartridge (1) is configured for conducting an alternative measurement using the second measuring section (12b, 12c), wherein particularly said alternative measurement is an optical measurement.

11. A System (200) comprising a sensor cartridge (1) according to one of the preceding claims and a sensor head (100) comprising a receiving section (101), particularly a slot (101), wherein the interface section (11) of the sensor cartridge (1) is configured to be connected to the receiving section (101) of the sensor head (100) so that the in- and outflow section (14) protrudes from the sensor head (100).

12. The system according to claim 11, wherein the sensor head (100) and/or the sensor cartridge (1) comprise a coupling device (205) configured to establish a fluid-tight connection with a measurement port (206) of a container (207) for accommodating the fluid (2), wherein particularly the container is a fluid line (207), or a fluid reservoir, or a reactor.

13. The system according to one of the claims 11 to 12, wherein the in- and outflow section (14) comprises the coupling device (205).

14. The system according to one of the claims 11 to 13, wherein the system further comprises a container (207) for accommodating the fluid (2) to be measured, wherein the sensor cartridge (1) is part of a closed fluid system comprising the container (207) and the sensor cartridge (1), and wherein the sensor head (100) is separated from said fluid system in a liquid tight fashion.

15. A method for inline measuring of a fluid using a system according to any one of claims 11 to 14, wherein the in- and outflow section of the sensor cartridge (1) being connected to the sensor head (100) is arranged in flow connection with a fluid (2) to be measured and a partial flow of the fluid (2) is pumped by the pump (13) into the sensor cartridge (1) via the inflow channel (15), wherein the partial flow is measured in the measuring channel (12) of the sensor cartridge (1) with help of the sensor head (100), and wherein the partial flow is discharged through the outflow channel (16) and united with the fluid (2).
